Europäisches Patentamt

European Patent Office

Office européen des brevets

(11) Publication number: **0 488 479 A1**

## EUROPEAN PATENT APPLICATION

(21) Application number: **91203116.8**

(22) Date of filing: **26.09.83**

(51) Int. Cl.5: **C12N 15/18**, C12P 21/02, C07K 13/00, A61K 37/36, C12N 1/21, //(C12N1/21, C12R1:19)

This application was filed on 28 - 11 - 1991 as a divisional application to the application mentioned under INID code 60.

(30) Priority: **27.09.82 GB 8227493**

(43) Date of publication of application:
**03.06.92 Bulletin 92/23**

(60) Publication number of the earlier application in accordance with Art.76 EPC: **0 104 920**

(84) Designated Contracting States:
**AT BE CH DE FR GB IT LI LU NL SE**

(71) Applicant: **BIOGEN, INC.**
**14 Cambridge Center**
**Cambridge Massachusetts 02142(US)**

(72) Inventor: **Movva, Nageswararao**
**10, Avenue Secheron**
**CH-1202 Geneva(CH)**
Inventor: **Schulz, Marie-Francoise**
**86A, Route Florissant**
**CH-1206 Geneva(CH)**

(74) Representative: **Bannerman, David Gardner et al**
**Withers & Rogers 4 Dyer's Buildings Holborn**
**London, EC1N 2JT(GB)**

(54) DNA sequences, recombinant DNA molecules and processes for producing swine growth hormone-like polypeptides.

(57) DNA sequences, recombinant DNA molecules and hosts transformed with them which produce polypeptides displaying a growth-enhancing biological activity of swine growth hormone. These polypeptides are useful in improving the rate of growth of and meat production and quality in swine.

EP 0 488 479 A1

## TECHNICAL FIELD OF INVENTION

This invention relates to DNA sequences, recombinant DNA molecules and processes for producing swine growth hormone and swine growth hormone-like polypeptides. More particularly, it relates to DNA sequences expressed in appropriate hosts and the products produced by that expression. The DNA sequences and recombinant DNA molecules of this invention are characterized in that they code for polypeptides having the growth enhancing biological activity of swine growth hormone. As will be appreciated from the disclosure to follow the DNA sequences, recombinant DNA molecules and processes of this invention may be used in the production of polypeptides useful as general anabolic agents in swine, most especially to increase the rate of growth, weight gain, and meat production in those animals.

## BACKGROUND ART

Swine growth hormone ("SGH") is a polypeptide hormone synthesized in and secreted from the anterior lobe of the pituitary. SGH is believed to be synthesized as a precursor protein (swine pregrowth hormone) and to be matured to swine growth hormone during secretion and release of the hormone into the blood stream. The partial amino acid sequence of swine growth hormone has been reported [J. B. Mills et al., "Cyanogen Bromide Cleavage And Partial Amino Acid Sequence Of Porcine Growth Hormone", J. Biol. Chem., 245, pp. 3407-15 (1970); A. E. Wilhelmi and J. B. Mills, "Studies On The Primary Structure Of Porcine Growth Hormone", in Growth and Growth Hormone, ed. A. Pecile and E. E. Muller, Amsterdam, Excerpta Medica Foundation, 1972, pp. 38-41. Intern. Congr. Ser. 244].

Growth hormones are normally produced throughout the life cycle, although apparently in higher amounts during the pre-adult period. These hormones are known to promote skeletal growth, nitrogen retention, protein synthesis and to affect glucose and lipid metabolism. Accordingly, growth hormones are recognized as general anabolic agents.

Growth hormones are somewhat species specific. However, a growth hormone from one species may be biologically active in another species lower in the evolutionary scale. Although the mechanism of growth hormone's activity is not well understood, it has been demonstrated that the administration of growth hormone markedly increases the rate of growth, weight gain, and meat production in animals. For example, in one test, the average rate of weight gain in pigs receiving daily injections of purified swine growth hormone was 2.26 pounds per day (as compared to an average weight gain of 2.19 lbs/day in control pigs). More importantly, the treated pigs consumed significantly less feed per day than the control pigs (7.03 lbs as compared to 8.40 lbs). In addition, the treated pigs displayed a marked improvement in carcass quality -- the carcasses of the growth hormone-treated pigs averaged 30.57 inches in length and had 1.40 inches of backfat, while those of the control group averaged 29.33 inches in length and had 1.77 inches of backfat. The chemical composition of the edible meat was also markedly improved in the growth hormone-treated animals -- 13.50% protein, 49.13% moisture and 36.76% fat -- as compared to the control group -- 10.8% protein, 39.43% moisture and 49.27% fat [E. J. Turman, "Some Effects Of Pituitary Anterior Growth Hormone On Swine", Thesis; Purdue University (April 1953)].

Unfortunately, the above-described improved growth and meat production in swine using swine growth hormone are not able to be widely realized because there is insufficient SGH available. Today, SGH is extracted from pituitary glands of swine. Plainly, this source is not nearly adequate to provide the needed commercial quantities of SGH.

Recent advances in molecular biology have made it possible to introduce the DNA coding for specific nonbacterial proteins into bacterial cells. In general, with DNA other than that prepared via chemical synthesis, the construction of such recombinant DNA molecules comprises the steps of producing a single-stranded DNA copy (cDNA) of a messenger RNA (mRNA) template coding for the desired protein; converting the cDNA to double-stranded DNA; linking the double-stranded cDNA to an appropriate site in an appropriate cloning vehicle to form a recombinant DNA molecule and transforming an appropriate host with that recombinant DNA molecule. Culturing of the transformed host may then permit the host to express the DNA sequence and to produce the desired protein.

The production of several non-bacterial proteins has been reported using recombinant DNA technology. These include bovine pre-growth hormone [e.g., W. L. Miller et al., "Molecular Cloning Of DNA Complementary To Bovine Growth Hormone mRNA", Journal Biological Chem., 255, pp. 7521-24 (1980), European patent application 47,600 and United Kingdom patent application 2,073,245A] and human pre-growth hormone [e.g., J. A. Martial et al., "Human Growth Hormone: complementary DNA Cloning And Expression In Bacteria", Science, 205, pp. 602-06 (1979) and European patent application 20,147].

None of these recombinant DNA processes, however, is directed as is this invention to swine growth hormone, to swine growth hormone-like polypeptides or to the marked improvement in meat production that would be attained if commercial quantities of swine growth hormone or swine growth hormone-like polypeptides were available.

## DISCLOSURE OF THE INVENTION

The present invention solves the problems referred to by isolating the DNA sequences that code for SGH or SGH-like polypeptides and by expressing those sequences in appropriate hosts to produce polypeptides displaying the growth enhancing biological activity of SGH.

By virtue of this invention, it is for the first time possible to obtain polypeptides displaying the activity of SGH in commercial quantities for use in diverse applications to increase the rate of growth and meat production in swine.

As will be appreciated from the disclosure to follow, the DNA sequences and recombinant DNA molecules of this invention are capable of directing the production, in an appropriate host, of swine growth hormone or swine growth hormone-like polypeptides, i.e., polypeptides displaying the growth-enhancing biological activity of SGH. The polypeptides of this invention are useful in compositions and methods for improving the growth rates of and meat production in swine.

It will accordingly be appreciated from the foregoing that a basic aspect of this invention is the preparation of a DNA sequence which is characterized in that it codes for a polypeptide displaying the growth-enhancing biological activity of SGH. Such DNA sequences comprise inserts selected from the group consisting of the DNA inserts of pBR322(Pst)/SGH-9D, pSGH-Δ7, pSGH-(Met-Phe), DNA sequences which hybridize to the foregoing inserts and which code for SGH-like polypeptides, and DNA sequences which on expression code for a polypeptide also coded for on expression by any of the foregoing DNA sequences or inserts.

## BRIEF DESCRIPTION OF THE DRAWINGS

Figure 1 is a schematic outline of one embodiment of a process of this invention for preparing a mixture of recombinant DNA molecules, some of which are characterized by inserted DNA sequences that code for the polypeptides of this invention.

Figure 2 is a schematic outline of two embodiments of clone screening processing to select the DNA sequences of this invention. In the first, an SGH-related cDNA sequence was selected by hybridization to a probe from bovine growth hormone. In the second, a probe constructed by digestion of the SGH-related cDNA was employed.

Figure 3 is an outline of the sequencing strategy employed to determine the nucleotide sequence of DNA sequence SGH-9D of this invention.

Figures 4-5 depict the nucleotide sequence of DNA sequence SGH-9D of this invention and the amino acid sequence corresponding thereto.

Figure 6 is a partial list of restriction sites determined by computer analysis of the nucleotide sequence displayed in Figures 4-5.

Figure 7 is a schematic outline of one embodiment of the construction of recombinant DNA molecule pSGH-Δ7, of this invention.

Figure 8 depicts the pertinent nucleotide and amino acid sequences of pBGH-Δ9(Ser), pSGH-9D and pSGH-Δ7.

Figures 9-10 are a schematic outline of one embodiment of the construction of recombinant DNA molecule pSGH-(Met-Phe) of this invention.

## BEST MODE OF CARRYING OUT THE INVENTION

In order that the invention herein described may be more fully understood, the following detailed description is set forth.

In the description the following terms are employed:

Nucleotide -- A monomeric unit of DNA or RNA consisting of a sugar moiety (pentose), a phosphate, and a nitrogenous heterocyclic base. The base is linked to the sugar moiety via the glycosidic carbon (1' carbon of the pentose). That combination of a base and a sugar is called a nucleoside. Each nucleotide is characterized by its base. The four DNA bases are adenine ("A"), guanine ("G"), cytosine ("C") and thymine ("T"). The four RNA bases are A, G, C and uracil ("U").

DNA Sequence -- A linear array of nucleotides connected one to the other by phosphodiester bonds between the 3' and 5' carbons of adjacent pentoses.

Codon -- A DNA sequence of three nucleotides (a triplet) which encodes through mRNA an amino acid, a translation start signal or a translation termination signal. For example, the nucleotide triplets TTA TTG, CTT, CTC, CTA and CTG encode for the amino acid leucine ("Leu"), TAG, TAA and TGA are translation stop signals and ATG is a translation start signal.

Reading Frame -- The grouping of codons during translation of mRNA into amino acid sequences. During translation the proper reading frame must be maintained. For example, the sequence GCTGGTTGTAAG may be translated in three reading frames or phases, each of which affords a different amino acid sequence:

$$\text{GCT GGT TGT AAG -- Ala-Gly-Cys-Lys}$$
$$\text{G CTG GTT GTA AG -- Leu-Val-Val}$$
$$\text{GC TGG TTG TAA G -- Trp-Leu-(STOP)}$$

Polypeptide -- A linear array of amino acids connected one to the other by peptide bonds between the $\alpha$-amino and carboxy groups of adjacent amino acids.

Genome -- The entire DNA of a cell or a virus. It includes, inter alia, the genes coding for the polypeptides of the substance, as well as operator, promoter and ribosome binding and interaction sequences, including sequences such as the Shine-Dalgarno sequences.

Gene -- A DNA sequence which encodes through its template or messenger RNA ("mRNA") a sequence of amino acids characteristic of a specific polypeptide.

Transcription -- The process of producing mRNA from a gene.

Translation -- The process of producing a polypeptide from mRNA.

Expression -- The process undergone by a DNA sequence or gene to produce a polypeptide. It is a combination of transcription and translation.

Plasmid -- A non-chromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance ($Tet^R$) transforms a cell previously sensitive to tetracycline into one which is resistant to it. A cell transformed by a plasmid is called a "transformant".

Phage or Bacteriophage -- Bacterial virus many of which consist of DNA sequences encapsidated in a protein envelope or coat ("capsid").

Cloning Vehicle -- A plasmid, phage DNA or other DNA sequence which is able to replicate in a host cell, which is characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance. A cloning vehicle is often called a vector.

Cloning -- The process of obtaining a population of organisms or DNA sequences derived from one such organism or sequence by asexual reproduction.

Recombinant DNA Molecule or Hybrid DNA --A molecule consisting of segments of DNA from different genomes which have been joined end-to-end outside of living cells and have the capacity to infect some host cell and be maintained therein.

Expression Control Sequence -- A sequence of nucleotides that controls and regulates expression of DNA sequences or genes when operatively linked to those sequences. They include the lac system, the trp system, major operator and promoter regions of phage λ, the control region of fd coat protein and other sequences known to control the expression of genes of prokaryotic or eukaryotic cells and their viruses or combinations thereof.

SGH -- Swine growth hormone.

SGH-Like Polypeptide -- A polypeptide displaying the growth-enhancing biological activity of SGH.

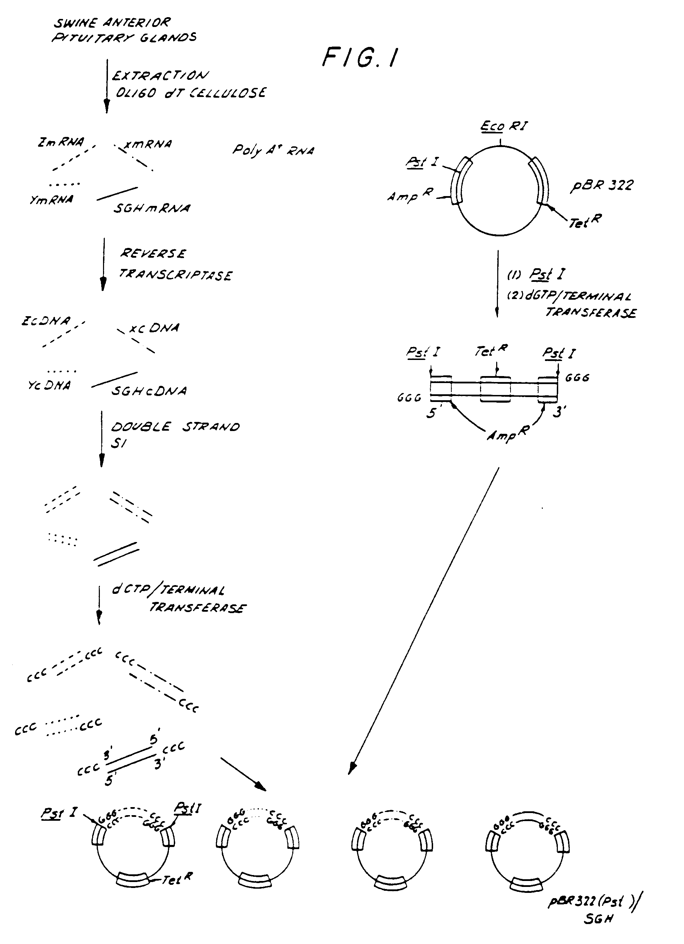

Referring now to Figure 1, we have shown therein a schematic outline of one embodiment of a process for preparing a mixture of recombinant DNA molecules, some of which are characterized by inserted DNA sequences that code for SGH or SGH-like polypeptides.

PREPARATION OF POLY A[+] RNA-CONTAINING SWINE GROWTH HORMONE mRNA (SGH-mRNA

We added 4-6 g swine anterior pituitary glands (Pelfreeze, Arkansas), frozen in dry ice, to a solution of 5 M guanidium thiocyanate, 50 mM sodium citrate, 0.5% sodium lauryl sarcosyl and 0.1 M $\beta$-mercaptoethanol (10 ml/g glands) [J. M. Chirgwin et al., Biochemistry, 18, pp. 5294-99 (1979)]. We then allowed the glands to thaw to 4°C in that solution and homogenized (Polytron) the mixture with six 20 sec bursts. After centrifugation of the homogenate (Sorvall, 6000 rpm, 5 min, room temperature), we layered 7.0 ml of the nucleic acid-containing supernatant onto 2.5 ml CsCl (5.7 M in 0.1 M EDTA) and covered it with paraffin oil. We then centrifuged the tube (SW 41, 30000 rpm, 18 h, 20°C) to pellet the RNA-containing fraction.

After discarding the supernatant and resuspending the total RNA-containing pellet in water, we extracted the aqueous solution twice with equal volumes of phenol-chloroform (1:1) and twice with equal volumes of ether. We then precipitated the RNA from the aqueous phase with 3 vol ethanol and collected the resulting precipitate by centrifugation. Yield of total RNA: 8 mg.

We resuspended 4 mg of the above-described total RNA in 1 ml Tris-HCl(10 mM, pH 7.5)-EDTA (1 mM) ("TE buffer") and heated the solution at 65°C for 10 min. After adding 2 M KCl to a final concentration of 0.5 M, we applied the mixture to an oligo-dT-cellulose column and washed the column with 0.5 M KCl to remove all of the ribosomal RNAs. Finally, we eluted the poly A$^+$ RNA that was bound to the column with 10 mM Tris-HCl(pH 7.5) at room temperature and pooled the poly A$^+$ RNA-containing fractions. We collected the RNA from that combined fraction, as before, by precipitation with ethanol and salts, centrifugation and resuspension in water. Yield of poly A$^+$ RNA: 200 $\mu$g.

To check the activity of our isolated poly A$^+$ RNA, we translated 2 $\mu$g of that poly A$^+$ RNA in vitro using a rabbit reticulocyte lysate $^{35}$S-methionine cell-free system (NEN) [H.R.B. Pelham and R. J. Jackson, Eur. J. Biochem., 67, pp. 247 et seq. (1976)] and assayed the product on a SDS/polyacrylamide gel via autoradiography. This assay demonstrated that our isolated poly A$^+$ RNA was translated to produce a polypeptide having a molecular weight that corresponded to the size expected for swine pre-growth hormone (about 24,000).

It should, of course, be understood that our poly A$^+$ RNA is a mixture of a large number of different RNAs (Figure 1). Except for the mRNA specific for SGH or SGH-like polypeptides, these RNAs are undesirable contaminants (Figure 1). Unfortunately, these contaminant RNAs behave similarly to SGH-mRNA throughout the remainder of the cloning process. Therefore, their presence in our poly A$^+$ RNA will result in a large number of useless clones and will present a complex screening problem to select a correct clone, i.e., one coding for SGH, from the many more numerous contaminants.

SYNTHESIS OF A cDNA MIXTURE CONTAINING SGH-cDNA

To prepare single-stranded DNA complements to our poly A$^+$ RNAs, we mixed 20 $\mu$l poly A$^+$ RNA (10 $\mu$g in H$_2$O) and 2 $\mu$l 0.15 M CH$_3$Hg and allowed the resulting solution to stand at room temperature for 15 min. We then added to the solution 10 $\mu$l oligo dT (1 $\mu$g/ml), 100 $\mu$l dNTPs (2.5 mM, i.e., to 0.5 mM final concentration), 20 $\mu$l $\alpha$-$^{32}$P-dATP (10 mCi/ml), 50 $\mu$l reverse transcriptase (16 units/$\mu$l, Life Sciences) and an equal volume of a buffer (100 mM Tris-HCl (pH 8.5), 20 mM MgCl$_2$, 140 mM KCl, 50 mM DTT in H$_2$O). The total volume of the solution was 404.4 $\mu$l. After incubating the solution for 90 min at 42°C, we heated it at 100°C for several minutes and centrifuged it (10000 G, 5 min.). The supernatant contained the single-stranded cDNAs complementary to our poly A$^+$ RNA. Yield of cDNA: 1.438 $\mu$g (via TCA precipitation of a small aliquot).

Again, it is to be understood that our single-stranded cDNA is actually a complex mixture of a large number of different cDNAs transcribed from the corresponding mRNAs present in our poly A$^+$ RNA mixture (Figure 1). Another factor also acts to complicate the cDNA mixture. Each mRNA species of the poly A$^+$ RNA may not be transcribed completely. Instead, a large variety of cDNAs may be produced from each mRNA species (not shown in Figure 1). Since each of these incomplete cDNAs behave similarly to the desired cDNA during the remainder of the process, their presence in the cDNA mixture only serves further to complicate the final clone screening process.

PREPARATION OF DOUBLE-STRANDED cDNA

To convert the above-prepared cDNA to double-stranded cDNA, we took one-half of our cDNA (0.7 $\mu$g) and added 50 $\mu$l Klenow (0.7 units/$\mu$l), 1.5 $\mu$g MgCl$_2$ (1 M) and an equal volume of DNA polymerase buffer (400 mM Tris-HCl (pH 6.9), 150 mM KCl, 1 mM dNTPs, 20 mM $\beta$-mercaptoethanol) and incubated the mixture for 17 h at 16°C and then for a further 30 min at 37°C. We then precipitated the DNA by adding 1/8 vol 2M sodium acetate (pH 5.5) and 2.5 vol ethanol and cooling to -70°C for 15 min. After collecting the precipitate by centrifugation (10000 g, 10 min), we washed the pellet with ethanol, and resuspended it in water and applied it to a Sephadex G-75 column. We eluted the DNA with 10 mM Tris-HCl-0.1 mM EDTA and pooled the DNA-containing fractions. Total vol of DNA-containing fractions: 377 $\mu$l.

We added 42 $\mu$l of a solution (2 M NaCl, 500 mM sodium acetate (pH 4.5), 10 mM zinc sulfate and 8 $\mu$l SI (1000 units/$\mu$l, Boehringer-Mannheim)) to the pooled DNA-containing fractions and allowed the solution to stand at 30°C for 30 min. We then extracted the mixture three times with equal volumes of phenol and then ether, applied the resulting aqueous phase to a Sephadex™ G-75 column and eluted the DNA with 5 mM KCl. We pooled the DNA-containing fractions and precipitated them with ethanol as before.

## CLONING OF DOUBLE-STRANDED DNA

A wide variety of host/cloning vehicle combinations may be employed in cloning and expressing double-stranded cDNA. In addition, within each specific cloning vehicle, various sites may be selected for insertion of the double-stranded cDNA. The particular selection for cloning and expression may be made by one of skill in the art without departing from the scope of this invention.

For our initial cloning we chose the bacterial plasmid pBR322 [F. Bolivar et al., "Construction And Characterization Of New Cloning Vehicles II. A Multi-Purpose Cloning System", Gene, pp. 95-113 (1977); J. G. Sutcliffe, "pBR322 Restriction Map Derived From The DNA Sequence Accurate DNA Size Markers Up To 4361 Nucleotide Pairs Long", Nucleic Acids Research, 5, pp. 2721-28 (1978)], the Pst I site therein [L. Villa-Komaroff et al., "A Bacterial Clone Synthesizing Proinsulin", Proc. Natl. Acad. Sci. USA, 75, pp. 3727-31 (1978)] and E. coli K12 MC1061 [M. Casadaban and S. N. Cohen, J. Mol. Biol., 138, pp. 179-207 (1980)].

### 1. Preparation of Pst I-Cleaved, dG-Tailed pBR322

We purified plasmid pBR322 on two consecutive CsCl gradients and digested 25 $\mu$g of it with Pst I endonuclease using standard conditions (Figure 1). We then added to the Pst-I cleaved plasmid 50 $\mu$l $H_2O$, 3 $\mu$l DTT (10 mM), 1.5 $\mu$l dGTP (10 mM), 10 $\mu$l gelatin (1 mg/ml), 10 $\mu$l terminal transferase buffer (10 mM $CoCl_2$, 1.4 M potassium cacodylate and 0.3 M Tris-HCl (pH 7.6)) and 0.9 $\mu$l terminal deoxynucleotidyl transferase (160 units/ ml, Ratcliff Biochemicals). After incubating the solution at 10°C for 13 min, we added 5 $\mu$l EDTA (250 mM) and extracted the resulting mixture twice with equal volumes phenolchloroform (1:1) and three times with equal volumes of ether. The aqueous phase was reserved for subsequent combination with dC-tailed double-stranded cDNA.

### 2. Preparation of dC-tailed cDNA

We added dC tails to our double-stranded cDNA by resuspending about 1/5 of the cDNA prepared previously (100 ng) in 25 $\mu$l $H_2O$, adding 50 $\mu$l $H_2O$, 3 $\mu$l DTT (10 mM), 1.5 $\mu$l dCTP (10 mM), 10 $\mu$l gelatin (1 mg/ml), 10 $\mu$l terminal transferase buffer (10 mM $CoCl_2$, 1.4 M potassium cacodylate, 0.3 M Tris-HCl (pH 7.6)) and 0.9 $\mu$l terminal deoxynucleotidyl transferase (160 units/ml, Ratcliff Biochemicals) (Figure 1). After incubating the mixture for 13 min at 10°C, we added 5 $\mu$l EDTA (250 mM) and extracted the resulting mixture twice with equal volumes phenolchloroform (1:1) and three times with equal volumes of ether. Again, we reserved the aqueous phase for combination with the dG-tailed, Pst-cleaved pBR322.

### 3. Annealing of dG-tailed pBR322 and dC-tailed cDNA

We combined 1 $\mu$l dG-tailed, Pst I-cleaved pBR322 (50 ng) and 2.5 $\mu$l dC-tailed cDNA (1 ng) in 5 $\mu$l of annealing buffer (1 M NaCl, 200 mM Tris-HCl (pH 7.6), 10 mM EDTA) and water to a final volume of 50 $\mu$l. After heating the mixture at 80°C for 3 min and at 45°C for a further 2 h, we allowed the temperature of the solution to drop to 4°C and allowed it to stand overnight.

Again, it should be understood that only a very few of the recombinant DNA molecules produced by such annealing contain a cDNA insert that is related to SGH (Figure 1).

### 4. Transfection of E. coli K12 MC1061 With Hybrids

We dialyzed the above-described annealed dG-tailed pBR322 and dC-tailed DNA in collodion bags (UH 100-25) against 1/10 annealing buffer (supra) for 2 h at 4°C to exclude any low molecular weight species. We then applied the mixture to 200 $\mu$l of E.coli K12 MC1061, previously made competent substantially as described in G. N. Buell et al., J. Biol. Chem., 254, pp. 927-983 (1979).

Since plasmid pBR322 includes the gene coding for tetracycline resistance and the gene coding for ampicillin resistance (Figure 1), and since that latter gene is inactivated if cDNA is inserted at the PstI site, colonies that have been transformed with recombinant DNA molecules having DNA inserts at the PstI site may be selected from colonies that have not been so transformed (Figure 1). We isolated about 10000 transformed colonies from our 1 ng of cDNA.

These colonies contain a variety of recombinant DNA molecules representing complete or partial copies of the mixture of RNAs in our poly A$^+$ RNA (Figure 1). The majority of these molecules do not contain an SGH-related DNA insert.

## SCREENING FOR A CLONE CONTAINING SGH-cDNA

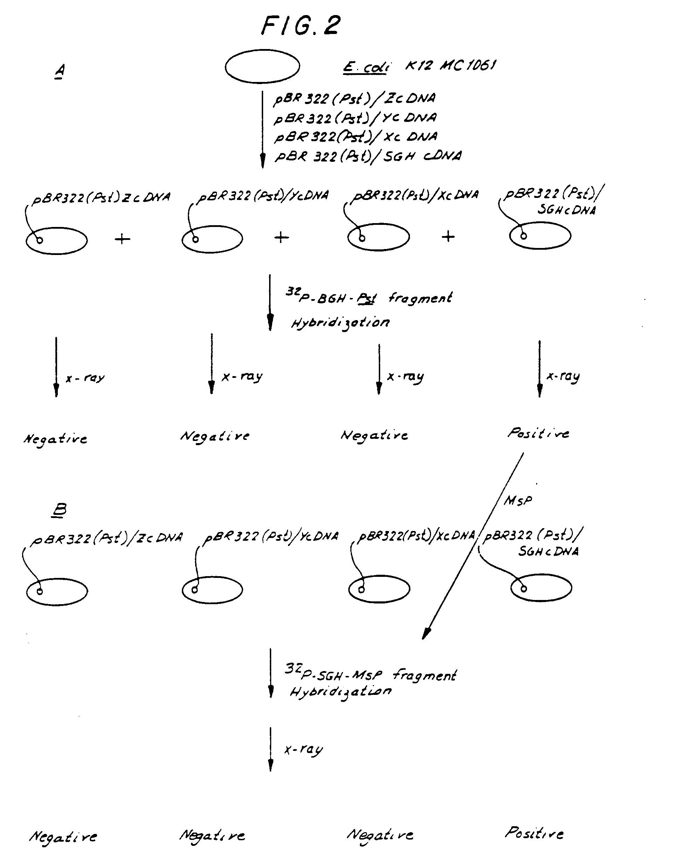

There are several approaches to screen a library of clones for a clone containing a particular recombinant DNA molecule, i.e. one containing an SGH-related DNA insert. For our initial clone screening we chose to use a 400 bp Pst I fragment of cDNA coding for bovine growth hormone [e.g., Miller et al., J. Biol. Chem., 255, pp. 7521-24 (1980)] (Figure 2). This sequence is also available by Pst I restriction of pBGH-(Met-Ala), deposited with the American Type Culture Collection on August 16, 1982 under ATCC accession number 39173.

We employed this probe for hybridization selection on a library of clones, prepared similarly to that described above (Figure 2). We first labelled the probe substantially as described by M. Grunstein and D. D. Hogness, "Colony Hybridization: A Method For The Isolation Of Cloned DNA's That Contain A Specific Gene," Proc. Natl. Acad. Sci. USA, 72, pp. 3961-65 (1975) and then employed it to screen our library of clones substantially as described infra using the SGH-Msp fragment probe.

From one of the hybridization positive clones we isolated a recombinant DNA molecule that had an inserted cDNA fragment that we determined by nucleotide sequencing to code for a portion of the amino acid sequence of SGH (by comparison with the reported partial amino acid sequence (supra)) (Figure 2).

We then prepared a 200 bp fragment of that positive cDNA (by digestion with Msp, see Figure 2) and labelled it (Grunstein and Hogness, supra) for use as a hybridization probe on our library of 10000 clones (Figure 2). For hybridization, we transferred the 10000 colonies onto nitrocellulose filters (Millipore) and incubated the filters overnight at 37°C. After lysing the colonies with 0.5 M NaOH for 7 min, we neutralized the filters with 1 M Tris-HCl (pH 7.5), 0.6 M NaCl (twice, 3 min each) and soaked the filters in 2X SSC (0.15 M NaCl-0.015 M sodium citrate (pH 7)). After air drying the filters, we baked them at 80°C in a vacuum oven for 2 h and then soaked them twice in 4X SSC (supra), 10X Denhardt's solution and 0.1% SDS for 2 h at 65°C. We then added 25000 $^{32}$P-counts/filter of the above-described 200 bp probe (boiled for 2 min and quickly cooled) and 4X SSC, 10X Denhardt's solution and 0.1% SDS, hybridized the filters at 65°C for 12-14 h, washed them in 4X SSC, 10X Denhardt's solution and 0.1% SDS at 65°C for 2 h, washed them with 2X SSC at 65°C for 30 min and dried them. After exposure on x-ray film for 1 day, we identified about 100 colonies that contained DNA inserts that hybridized to our probe.

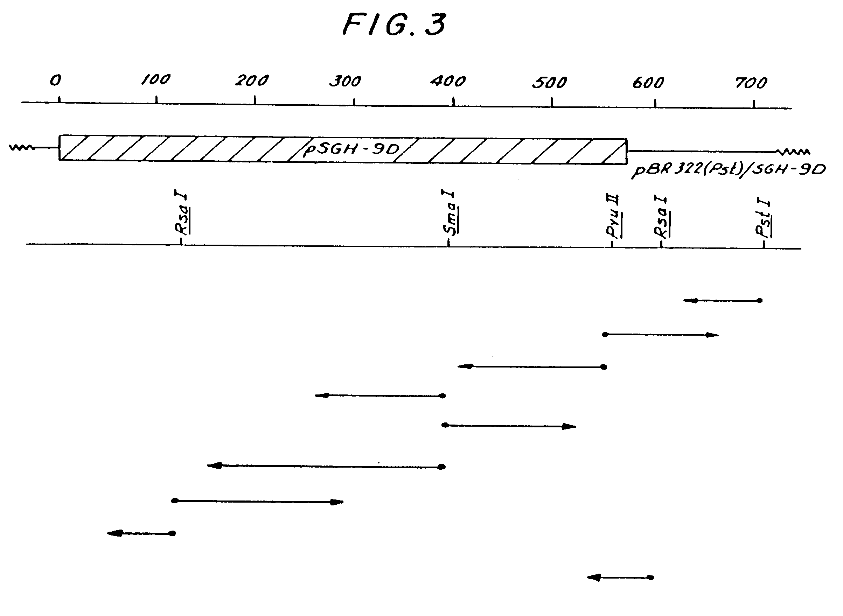

We chose 40 of these positive clones for restriction analysis and sizing (Pvu I/Bgl I) (Figure 3). From this analysis we determined that one of these clones had a DNA insert of about 750 bp. We designated this clone E. coli K12 MC1061 (pBR322(Pst)/SGH-9D), its recombinant DNA molecule pBR322(Pst)/SGH-9D and its insert SGH-9D. This nomenclature indicates that the clone comprises E. coli K12 MC1061 transformed with a recombinant DNA molecule comprising pBR322 and SGH-9D, insert SGH-9D being located at the Pst I site in pBR322 (Figure 3).

## NUCLEOTIDE SEQUENCE DETERMINATION OF INSERT SGH-9D

Because we predicted that a DNA sequence coding for SGH and its putative signal sequence would comprise about 648 nucleotides, we selected insert SGH-9D for analysis by restriction mapping and nucleotide sequence determination.

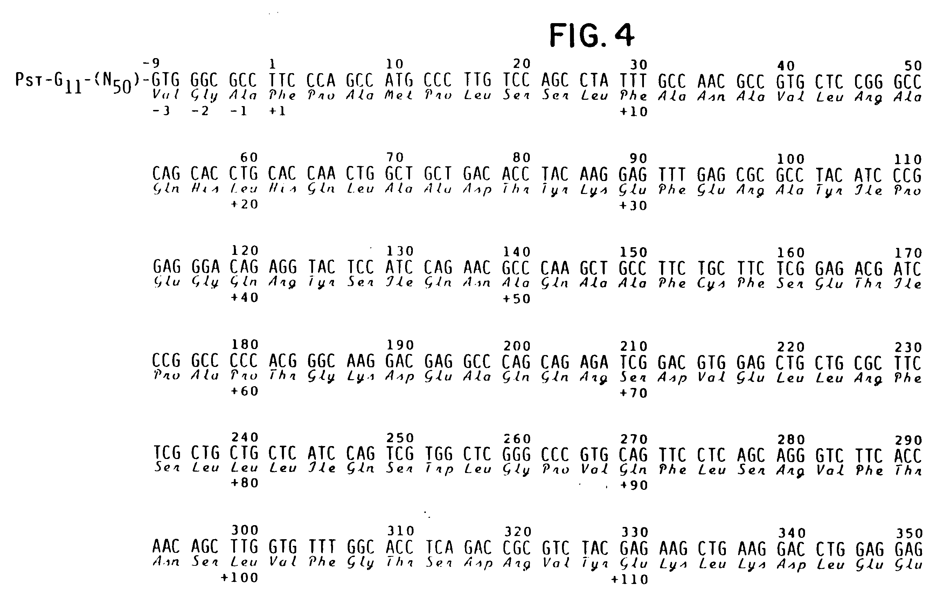

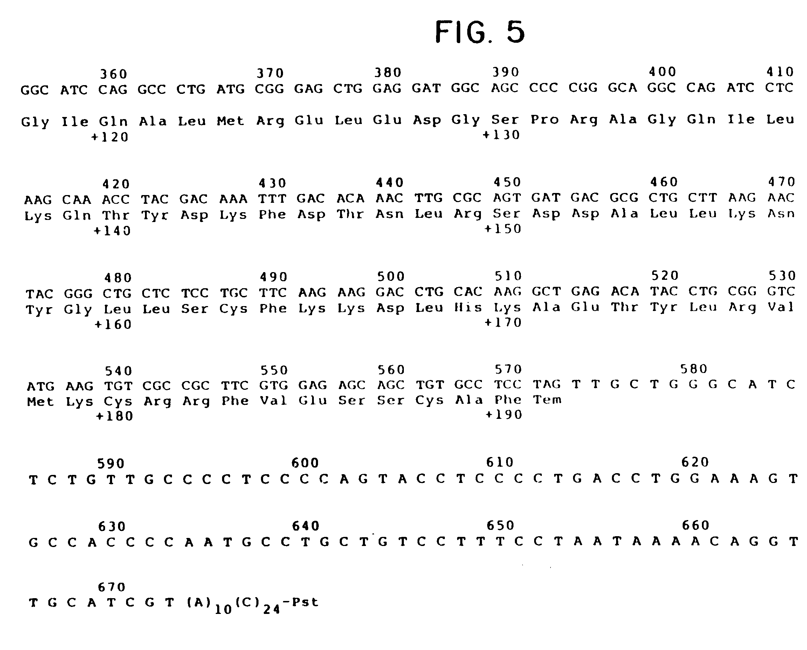

Referring now to Figure 3, we have depicted therein the strategy which we employed to determine the nucleotide sequence of SGH-9D. For DNA sequencing, we employed a method substantially as described by A. M. Maxam and W. Gilbert, "A New Method For Sequencing DNA", Proc. Natl. Acad. Sci. USA., 74, pp. 560-64 (1977). Most stretches of insert SGH-9D were sequenced from both strands and most restriction sites which served as labelled termini (the black dots in Figure 3) were sequenced using fragments spanning them. The nucleotide sequence thus obtained is depicted in Figures 4-5.

Referring now to Figures 4-5, the insert is flanked by 11 G residues at the 5' end and by 10A residues (probably reflecting the poly A$^+$ terminus of the mRNA) followed by 24C residues at the 3' terminus. For reference the insert is numbered from the first nucleotide following the dG tail to the last nucleotide before the poly A$^+$ residues.

By comparison with the partial amino acid sequence reported for SGH, it appears that nucleotides 1-570 code for SGH, with nucleotides 571-573 being a termination codon. Therefore, it appears that SGH is a protein of 190 amino acids. From the sequence it also appears that a swine pre-growth hormone exists. However, we have not yet determined the actual length or composition of the putative signal or pre-sequence of SGH because SGH-9D appears not to include all of the 5' coding and non-coding end of swine pre-growth hormone. We have designated the first three amino acids of the putative signal sequences as acids -1, -2 and -3 (Figures 4-5). SGH-9D does appear to include all of the 3' non-coding region because of the location of the poly A$^+$ residues.

It should, of course, be understood that the selection of clones containing the entire 5, coding and non-coding region and the identification of the complete pre-sequence of swine pre-growth hormone may be done by those of skill in the art without departing from the scope of our invention. For example, the positive clones that we have identified may be screened substantially as before using a probe taken from the 5' end of insert SGH-9D. For example, such a probe may be prepared by digesting SGH-9D with Nar I and isolating the fragment between nucleotides -5 and -60 (Figure 4). This fragment may then be labelled, as before, and used to select a clone containing a more complete 5' portion of SGH. That clone, if it does not also include the complete 3' end of SGH-9D may then be combined with all or part of SGH-9D through a common restriction site, as is well known in the art, to produce a single DNA sequence comprising the entire coding and non-coding regions of SGH.

It should be understood that the protein structure set forth in Figures 4-5 does not exclude modifications at the gene level, i.e., single or multiple base substitutions, which also provide a protein having the amino acid sequence depicted in Figures 4-5.

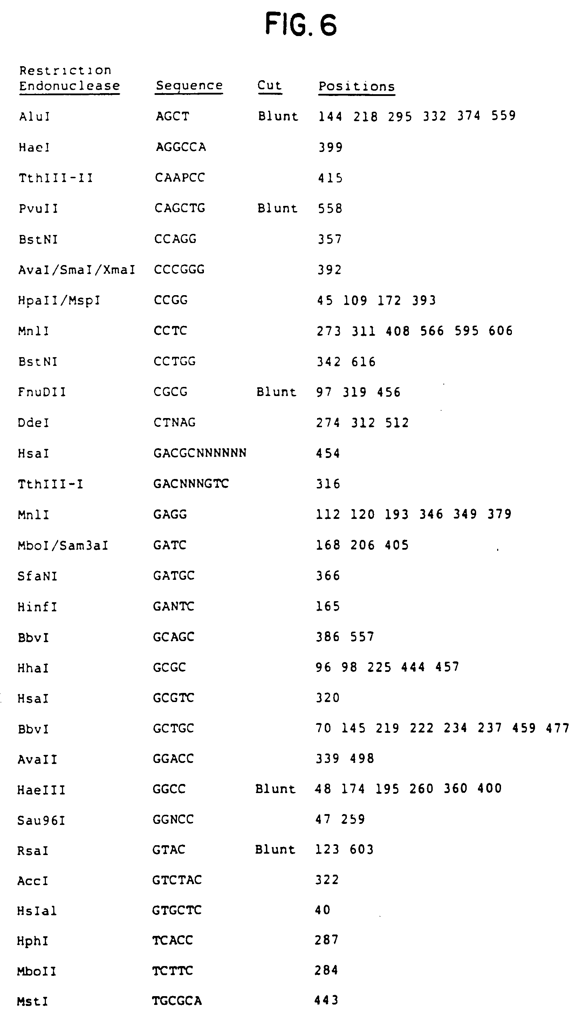

We have also analyzed the nucleotide sequence of SGH-9D by computer to determine the identity and location of some of the restriction endonuclease sites therein. The results of our analysis are displayed in Figure 6.

SYNTHESIS OF A SGH-LIKE POLYPEPTIDE

The level of production of a protein is governed by two major factors: the number of copies of its gene within the cell and the efficiency with which those gene copies are transcribed and translated. Efficiency of transcription and translation (which together comprise expression) is in turn dependent upon nucleotide sequences, normally situated ahead of the desired coding sequence. These nucleotide sequences or expression control sequences define, inter alia, the location at which RNA polymerase interacts to initiate transcription (the promoter sequence) and at which ribosomes bind and interact with the mRNA (the product of transcription) to initiate translation. Not all such expression control sequences function with equal efficiency. It is thus of advantage to separate the specific coding sequences for a desired protein from their adjacent nucleotide sequences and to fuse them instead to other expression control sequences so as to favor higher levels of expression. This having been achieved, the newly-engineered DNA fragment may be inserted into a multicopy plasmid or a bacteriophage derivative in order to increase the number of gene copies within the cell and thereby further to improve the yield of expressed protein.

A wide variety of host-expression control sequence vector combinations may, therefore, be employed in producing SGH-like polypeptides in accordance with the processes of this invention. For example, useful vectors may consist of segments of chromosomal, non-chromosomal and synthetic DNA sequences, such as various known bacterial plasmids from E. coli including col El, pCRI, pBR322 and their derivatives, wider host range plasmids e.g., RP4, phage DNA, e.g. the numerous derivatives of phage λ and vectors derived from combinations of the above, such as vectors that include a portion of pBR322, a portion of phage λ and a synthetic portion. Useful hosts may include bacterial hosts such as strains of E. coli, e.g., E. coli K12 MC1061, E. coli HB 101, E. coli X1776, E. coli X2282, E. coli MRCI and strains of Pseudomonas, Bacillus subtilis, Bacillus stearothermophiius and other bacilli, yeasts and other fungi, animal or plant hosts such as animal (including human) or plant cells in culture or other hosts. Useful expression control sequences may include the operator, promoter and ribosome binding and interaction sequences (including sequences such as the Shine Dalgarno sequences) of the lactose operon of E. coli ("the lac system"), the corresponding sequences of the tryptophan synthetase system of E. coli ("the trp system"), the major operator and promoter regions of phage λ ($O_LP_L$ and $O_RP^I_R$), the control region of the phage fd coat protein, or other sequences which control or aid the expression of genes of prokaryotic or eukaryotic cells and their viruses or various combinations of them.

Of course, not all host-expression control sequence-vector combinations may be equally efficient with a particular DNA coding sequence. However, as described in this invention and giving due consideration to biosafety, the sites available in the SGH-coding DNA sequences of this invention for particular constructions, the size of the SGH-like polypeptides to be expressed, the susceptibility of those polypeptides to proteolytic degradation by host cell enzymes, the possible contaminations of those polypeptides by host cell proteins difficult to remove during purification, the expression characteristics of SGH and SGH-like coding sequences, such as structure of the DNA sequence and the location of the start and stop codons with respect to the expression control sequences and other factors recognized by those skilled in the art, an appropriate combination may be selected.

There are also various methods known in the art for inserting a DNA sequence and expression control sequence into a vector. These include, for example, direct ligation, synthetic linkers, exonuclease and polymerase-linked repair reactions followed by iigation, or extension of the DNA strand with DNA polymerase and an appropriate single-stranded template followed by ligation. Any of these may be chosen for the synthesis of SGH-like polypeptides of this invention.

It should also be understood that the actual DNA sequence expressed in a chosen host-expression control sequence-vector combination may result in products which are not identical to mature SGH. For example, the DNA sequence expressed might code for mature SGH plus a methionine or part or all of the putative signal sequence of SGH or other amino acids unrelated to SGH. The DNA sequence expressed might alternatively code for only a part or parts of SGH alone or together with methionine or other amino acids. Both situations are encompassed by this invention. For example, a host transformed with a nucleotide sequence coding for a swine pregrowth hormone or f-met-SGH might produce that SGH-like compound alone or fused to other amino acids or it might secrete a mature SGH product. All that is necessary is that the product, either after isolation from the fermentation culture or after conventional treatment such as cleavage, synthetic linking or other well-known processes displays a growth-enhancing biological activity of SGH.*

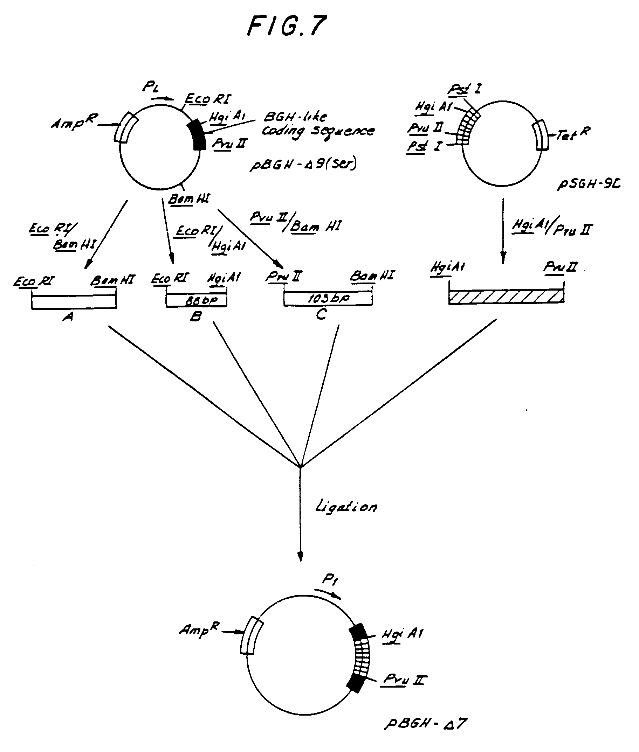

In the present synthesis, we chose the following construction to express a swine-growth hormone-like polypeptide: SGH-Δ7. We prepared this recombinant DNA molecule as depicted in Figure 7.

We first isolated pBGH-Δ9(Ser) from E.coli K12 λ(pBGH-Δ9(Ser)), a gift of Gary Buell and a culture deposited with the American Type Culture Collection on August 16, 1982 under accession number 39174, using the plasmid isolation method, substantially as described by R. D. Klein et al., Plasmid, 3, pp. 88-91 (1980). We then cleaved this plasmid with restriction endonucleases EcoRI and BamHI and isolated the EcoRI-BamHI fragment (Fragment A) (Figure 7). We also cleaved the same plasmid with EcoRI and HgiA₁ and isolated the about 88 base pair EcoRI-HgiA₁ fragment (Fragment B) (Figure 7). We also cleaved the same plasmid with PvuII and BamHI and isolated about 103 base pair PvuII-BamHI fragment (Fragment C) (Figure 7).

We then restricted pSGH-9D, described above, with HgiA₁ and PvuII using substantially the same conditions and isolated that part of the SGH coding sequence extending from the HgiA₁ Site (GTGCT↓C) to the PvuII site (CAG↓CTG) (Figures 7-8). We then ligated this fragment with the three fragments (A, B and C) previously prepared from pBGH-Δ9(Ser) using standard conditions and T4 DNA ligase at 16°C overnight.

Because of the similarities between the BGH coding sequence of pBGH-Δ9(Ser) and pSGH-9D and the degeneracies of the genetic code, the recombinant DNA molecule that resulted from the above-described ligation encodes an SGH-like polypeptide that following an f-Met start is missing its first 7 amino acids (Figures 7-8).

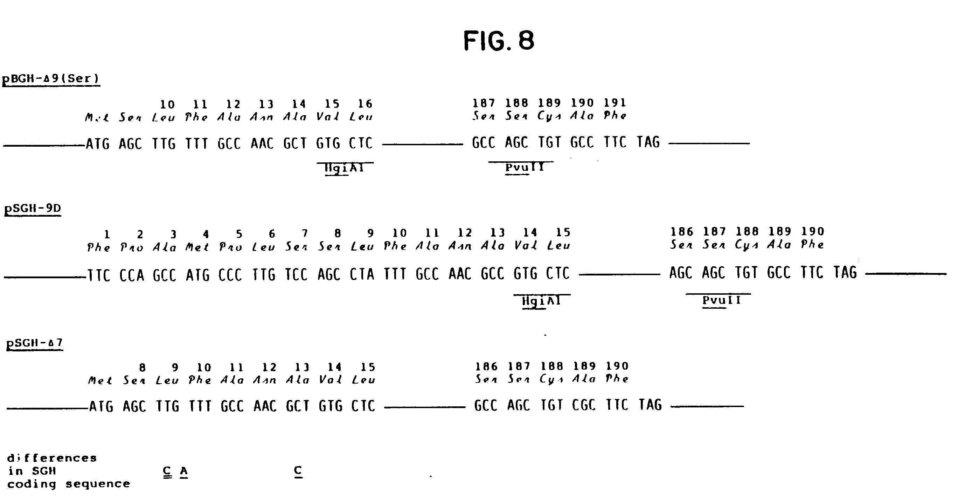

The SGH coding sequence that characterizes this molecule has 3 nucleotides that are different from those encoding SGH in PSGH-9D (Figures 4-5). Two of these differences arise from the differences between the BGH coding sequence and the SGH coding sequence (A and C, Figure 8). The third difference arises from the particular construction employed to produce pBGH-Δ9(Ser) ( C̲, Figure 8). None of these nucleotide differences changes the amino acid sequence of the SGH-like polypeptide encoded for by the inserted DNA sequence of pSGH-Δ7.

We then transformed 200 μl of competent E.coli K12 λ (a gift of Walter Fiers) in the presence of 10 μl 0.1 M $MgCl_2$ by chilling the cells in ice for 15 min, incubating them at 42°C for 2 min and incubating them at room temperature for 10 min. After adding 2 ml L-broth, we grew the cells at 37°C for 1 h and plated a 200 μl aliquot onto petri plates, containing L-broth supplemented with 50 μg/ml ampicillin. We grew our colonies at 37°C for 16 h and randomly selected 24 clones resistant to ampicillin.

We grew up cultures of each of these clones in 5 ml of L-broth, supplemented with 50 μg/ml ampicillin, for 12 h at 37°C and purified plasmid DNA from 1 ml of cells from each culture by the miniprep method

* It should also be understood that the f-Met or other non-SGH-related amino acids or non-activity essential amino acids of SGH itself may be removed chemically or enzymatically before use of the product. They may also be cleaved by the host cell during expression.

described by Klein et al., supra. We then sized the EcoRI-BamHI inserts in the 24 plasmids by EcoRI-BamHI restriction and polyacrylamide gel fractionation. We selected 9 fragments that had appropriately sized inserts. We then transformed E.coli MC1061, substantially as described in G. N. Buell et al., J. Biol. Chem., 254, pp. 9279-83 (1979), with one of these clones, designated pSGH-Δ7. We also transformed the cells with pcl857 (a gift of W. Fiers).*We selected cells transformed with pSGH-Δ7 and pcl857 by growing the colonies for 16 h at 30°C in L-Broth, supplemented with ampicillin (50 μg/ml) and Kanamycin (40 μg/ml).

We then randomly picked ampicillin and kanamycin-resistant colonies and grew them overnight in 5 ml L-Broth, supplemented as before with ampicillin and kanamycin, at 30°C. We then diluted the cultures with 25 ml L-Broth at 42°C and allowed the cells to grow at 42°C for 2 h (O.D.=1).

To isolate the SGH-like polypeptides produced by the transformed cells, we took 1 ml of cells and spun them down (8000 rpm, 4 min), added 50 μl Laemmli buffer (1% SDS) [Laemmli, Nature, 227, pp. 681 et seq. (1970)], boiled the mixture for 15 min, and again centrifuged the cells (8000 rpm, 4 min) to remove the cellular debris. We assayed the supernatant by SGH competition radioimmunoassay using rabbit antisera to authentic BGH. Yield: SGH activity (mg/l) 32; SGH-like molecules per cell $1.6 \times 10^6$.

Therefore, the DNA sequences and recombinant DNA molecules of this invention permit the production of SGH-like polypeptides in high yield. Moreover, the sequences, molecules and processes of this invention permit the production of novel SGH-like polypeptides, useful after purification using conventional methods, as general anabolic agents in swine, most especially to increase the rate of growth, weight gain and meat production in those animals.

While the above-described embodiment of a process for producing a SGH-like polypeptide in accordance with this invention affords a SGH-like polypeptide, missing its first 7 amino acids and including an f-Met, as compared to authentic SGH, other constructions may be made by those of skill in the art to produce other SGH-like polypeptides without departing from the scope of this invention. For example, a construction may be made wherein an ATG start codon or an ATG start codon and other codons is fused directly to the TTC codon coding for the first amino acid of authentic SGH. Such construction would enable the production of a mature SGH fused to an f-Met or through other amino acids to an f-Met.*We however prefer to employ pSGH-Δ7 in E.coli HB101 to produce the SGH-like polypeptides of this invention because much higher yields are obtained.

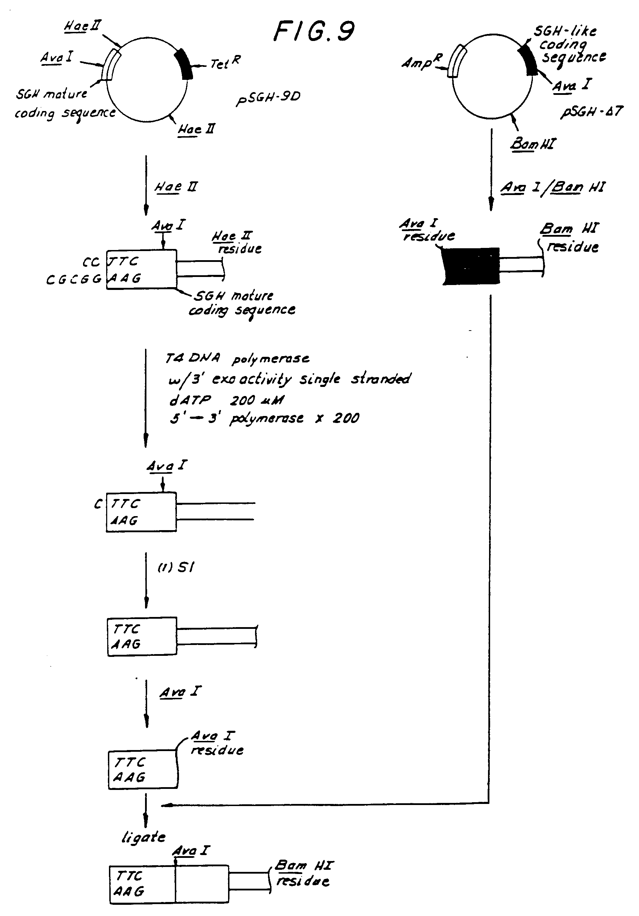

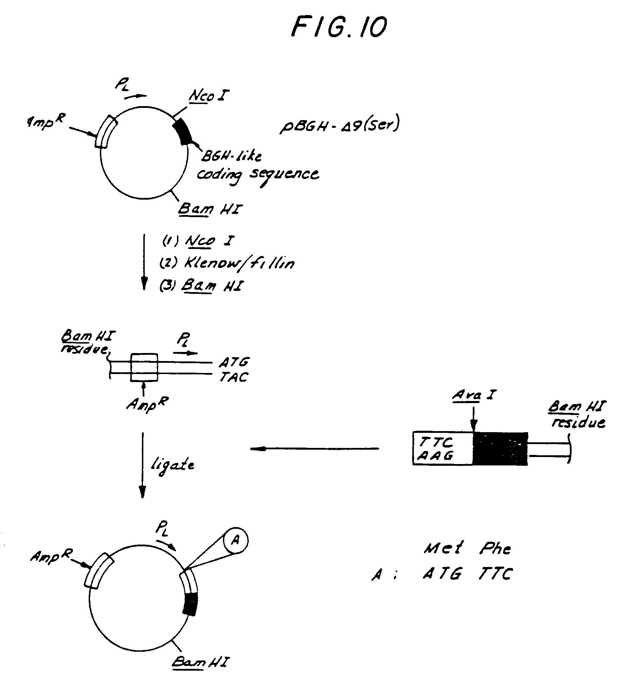

One method useful for preparing a construction that encodes f-Met-mature SGH is depicted in Figures 9-10. In that embodiment, we have restricted pSGH-9D with HaeII, removed the nucelotides remaining in front of the TTC codon encoding amino acid 1 of mature SGH and restricted the remaining fragment with AvaI to isolate a 5' coding end of mature SGH beginning with TTC. We then combined this fragment with an AvaI-BamHI fragment from pSGH-Δ7 encoding the 3' coding end of mature SGH. We then inserted that fragment into a cloning vector derived from pBGH-Δ9(Ser), described previously, such that the TTC codon was blunt-ended to an ATG codon. Accordingly, this construction (pSGH-(Met-Phe)) enables the production of f-Met-mature SGH in appropriate hosts. We have also prepared a Δ3-SGH using techniques as described above.

It should be understood that not all such constructions may be equally effective in producing the desired SGH-like polypeptides of this invention. However, one of skill in the art may readily select a construction and polypeptide appropriate for a particular expression system, host and application using the methods and assays described herein.

The above described SGH-like polypeptides after purification may be employed to treat swine using means and methods conventionally known for the administration of growth hormones to animals [e.g., E. J. Turman, supra] to improve their rate of growth and meat production. For example, SGH-Δ7, produced as above, has shown a weight gain enhancing activity similar to natural SGH in rat tests.

Microorganisms and recombinant DNA molecules prepared by the processes of this invention are exemplified by cultures deposited in the culture collection American Type Culture Collection, in Rockville, Maryland, on September 15, 1982, and identified as SGH-A and B:

A: E.coli K12 MC1061 (pSGH-9D)

B: E.coli K12 λ (pSGH-Δ7)

* pcl857 is a small plasmid that carries a temperature sensitive $P_L$ repressor and the gene coding for kanamycin resistance.

* It should be understood that any amino acid which is not part of mature SGH or non-activity essential amino acids of SGH itself may be removed during expression or subsequent thereto before the polypeptide is used to treat animals.

These cultures were assigned accession numbers ATCC 39191-39192, respectively.

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than the specific embodiments which have been presented hereinbefore by way of example.

**Claims**

1. A recombinant DNA molecule characterized by a DNA sequence encoding a polypeptide displaying the biological activity of swine growth hormone, said DNA sequence being selected from the group consisting of:
   a) the DNA insert of pSGH-9D,
   b) DNA inserts having a 5' terminal deletion from the DNA sequence coding for mature swine growth hormone and which code on expression for a polypeptide displaying the biological activity of swine growth hormone, and
   c) DNA sequences which are degenerate as a result of the genetic code to any of the foregoing DNA inserts and which code on expression for a polypeptide displaying the biological activity of swine growth hormone.

2. The recombinant DNA molecule according to claim 1, characterized by an expression control sequence, said expression control sequence being operatively linked to said DNA sequence.

3. The recombinant DNA molecule according to claim 2, characterized in that said expression control sequence is selected from the group consisting of sequences that control the expression of genes of prokaryotic or eukaryotic cells and their viruses and combinations thereof.

4. The recombinant DNA molecule according to claim 3, characterized in that said expression control sequence is selected from the group consisting of the lac system, the trp system, the major operator and promoter regions of phage λ and the control region of fd coat protein.

5. A polypeptide displaying the biological activity of swine growth hormone characterized in that it is coded for by a DNA sequence selected from the group consisting of:
   a) the DNA insert of pSGH-9D,
   b) DNA inserts having a 5' terminal deletion from the DNA sequence coding for mature swine growth hormone and which code on expression for a polypeptide displaying the biological activity of swine growth hormone, and
   c) DNA sequences which are degenerate as a result of the genetic code to the DNA insert of pSGH-(Met-Phe), the DNA insert of pSGH-Δ7, or to any of the foregoing DNA inserts and which code on expression for a polypeptide displaying the biological activity of swine growth hormone.

6. A DNA sequence characterized in that it codes for a polypeptide displaying the biological activity of swine growth hormone, said DNA sequence being selected from the group consisting of:
   a) the DNA inserts of pSGH-(Met-Phe), pSGH-Δ7 and pSGH-9D,
   b) DNA inserts having a 5' terminal deletion from the DNA sequence coding for mature swine growth hormone and which code on expression for a polypeptide displaying the biological activity of swine growth hormone, and
   c) DNA sequences which are degenerate as a result of the genetic code to any of the foregoing DNA inserts and which code on expression for a polypeptide displaying the biological activity of swine growth hormone.

7. A unicellular host transformed with a recombinant DNA molecule characterized by a DNA sequence encoding a polypeptide displaying the biological activity of swine growth hormone, said DNA sequence being operatively linked to an expression control sequence in said recombinant DNA molecule and being selected from the group consisting of:
   a) the DNA inserts of pSGH-(Met-Phe), pSGH-Δ7 and pSGH-9D,
   b) DNA inserts having a 5' terminal deletion from the DNA sequence coding for mature swine growth hormone and which code on expression for a polypeptide displaying the biological activity of swine

EP 0 488 479 A1

growth hormone, and

c) DNA sequences which are degenerate as a result of the genetic code to any of the foregoing DNA inserts and which code on expression for a polypeptide displaying the biological activity of swine growth hormone.

**8.** The unicellular host according to claim 7, said host being selected from the group consisting of E.coli, Pseudomonas, Bacillus, Streptomyces, fungi, animal cells, plant cells and human cells in tissue culture.

**9.** A process for producing a polypeptide displaying the biological activity of swine growth hormone characterized by the step of culturing a unicellular host transformed with a recombinant DNA molecule characterized by a DNA sequence, said DNA sequence being operatively linked to an expression control sequence in said recombinant DNA molecule and being selected from the group consisting of:

a) the DNA insert of pSGH-9D,

b) DNA inserts having a 5' terminal deletion from the DNA sequence coding for mature swine growth hormone and which code on expression for a polypeptide displaying the biological activity of swine growth hormone, and

c) DNA sequences which are degenerate as a result of the genetic code to the DNA insert of pSGH-(Met-Phe), the DNA insert of pSGH-Δ7, or to any of the foregoing DNA inserts and which code on expression for a polypeptide displaying the biological activity of swine growth hormone.

**10.** A composition for treating animals characterized by the polypeptide according to claim 5.

**Claims for the following Contracting State: AT**

**1.** A process for producing a recombinant DNA molecule comprising the step of introducing into a cloning vehicle a DNA sequence encoding a polypeptide displaying the biological activity of swine growth hormone, said DNA sequence being selected from the group consisting of:

a) the DNA insert of pSGH-9D,

b) DNA inserts having a 5' terminal deletion from the DNA sequence coding for mature swine growth hormone and which code on expression for a polypeptide displaying the biological activity of swine growth hormone, and

c) DNA sequences which are degenerate as a result of the genetic code to any of the foregoing DNA inserts and which code on expression for a polypeptide displaying the biological activity of swine growth hormone.

**2.** The process according to claim 1, characterized in that said DNA sequence is operatively linked to an expression control sequence in said recombinant DNA molecule and is expressed to produce a polypeptide displaying the biological activity of swine growth hormone when a unicellular host transformed with said recombinant DNA molecule is cultured.

**3.** The process according to claim 2, characterized in that said expression control sequence is selected from the group consisting of sequences that control the expression of genes of prokaryotic or eukaryotic cells and their viruses and combinations thereof.

**4.** The process according to claim 3, characterized in that said expression control sequence is selected from the group consisting of the lac system, the trp system, the major operator and promoter regions of phage λ and the control region of fd coat protein.

**5.** The process according to claim 2, characterized in that said molecule is contained in the transformed E.coli K12 MC1061 strain identified by accession number ATCC 39191.

**6.** A process for transforming a unicellular host comprising the step of introducing into said host a recombinant DNA molecule produced by the process according to claim 2.

**7.** The process according to claim 6, characterized in that said transformed host is the transformed E.coli K12 MC1061 strain identified by accession number ATCC 39191.

**8.** A process for producing a polypeptide displaying the biological activity of swine growth hormone

12

comprising the step of culturing a unicellular host transformed according to the process of claim 6.

9. A process for producing a polypeptide displaying the biological activity of swine growth hormone comprising the step of culturing a unicellular host transformed by a recombinant DNA molecule characterized by a DNA sequence encoding a polypeptide displaying the biological activity of swine growth hormone, said DNA sequence being operatively linked to an expression control sequence in said molecule and being selected from the group consisting of:
   a) the DNA insert of pSGH-9D,
   b) DNA inserts having a 5' terminal deletion from the DNA sequence coding for mature swine growth hormone and which code on expression for a polypeptide displaying the biological activity of swine growth hormone, and
   c) DNA sequences which are degenerate as a result of the genetic code to the DNA insert of pSGH-(Met-Phe), the DNA insert of pSGH-Δ7, or to any of the foregoing DNA inserts and which code on expression for a polypeptide displaying the biological activity of swine growth hormone.

FIG. 1

# FIG. 2

_A_

E. coli K12 MC 1061

pBR 322 (Pst)/Zc DNA
pBR 322 (Pst)/Yc DNA
pBR 322 (Pst)/Xc DNA
pBR 322 (Pst)/SGH cDNA

pBR322(Pst)Zc DNA    pBR322(Pst)/YcDNA    pBR322(Pst)/XcDNA    pBR322(Pst)/SGHcDNA

+      +      +

$^{32}$P-BGH-Pst fragment
Hybridization

x-ray      x-ray      x-ray      x-ray

Negative      Negative      Negative      Positive

MsP

_B_

pBR322(Pst)/Zc DNA    pBR322(Pst)/YcDNA    pBR322(Pst)/XcDNA    pBR322(Pst)/SGHcDNA

$^{32}$P-SGH-MsP fragment
Hybridization

x-ray

Negative      Negative      Negative      Positive

15

FIG. 3

0   100   200   300   400   500   600   700

pSGH-9D

pBR 322(Pst)/SGH-9D

Rsa I    Sma I    Pvu II   Rsa I    Pst I

EP 0 488 479 A1

# FIG. 4

```
                -9              1               10              20              30              40              50
PsT-G11-(N50)-GTG GGC GCC TTC CCA GCC ATG CCC TTG TCC AGC CTA TTT GCC AAC GCC GTG CTC CGG GCC
              Val Gly Ala Phe Pro Ala Met Pro Leu Ser Ser Leu Phe Ala Asn Ala Val Leu Arg Ala
              -3  -2  -1  +1                                    +10

                60              70              80              90              100             110
             CAG CAC CTG CAC CAA CTG GCT GCT GAC ACC TAC AAG GAG TTT GAG CGC GCC TAC ATC CCG
             Gln His Leu His Gln Leu Ala Ala Asp Thr Tyr Lys Glu Phe Glu Arg Ala Tyr Ile Pro
                 +20                             +30

                120             130             140             150             160             170
             GAG GGA CAG AGG TAC TCC ATC CAG AAC GCC CAA GCT GCC TTC TGC TTC TCG GAG ACG ATC
             Glu Gly Gln Arg Tyr Ser Ile Gln Asn Ala Gln Ala Ala Phe Cys Phe Ser Glu Thr Ile
                 +40                         +50

                180             190             200             210             220             230
             CCG GCC CCC ACG GGC AAG GAC GAG GCC CAG CAG AGA TCG GAC GTG GAG CTG CTG CGC TTC
             Pro Ala Pro Thr Gly Lys Asp Glu Ala Gln Gln Arg Ser Asp Val Glu Leu Leu Arg Phe
                 +60                             +70

                240             250             260             270             280             290
             TCG CTG CTG CTC ATC CAG TCG TGG CTC GGG CCC GTG CAG TTC CTC AGC AGG GTC TTC ACC
             Ser Leu Leu Leu Ile Gln Ser Trp Leu Gly Pro Val Gln Phe Leu Ser Arg Val Phe Thr
                 +80                                     +90

                300             310             320             330             340             350
             AAC AGC TTG GTG TTT GGC ACC TCA GAC CGC GTC TAC GAG AAG CTG AAG GAC CTG GAG GAG
             Asn Ser Leu Val Phe Gly Thr Ser Asp Arg Val Tyr Glu Lys Leu Lys Asp Leu Glu Glu
                 +100                            +110
```

EP 0 488 479 A1

# FIG. 5

```
        360                 370                 380                 390                         400              410
GGC ATC CAG GCC CTG ATG CGG GAG CTG GAG GAT GGC AGC CCC CGG GCA GGC CAG ATC CTC

Gly Ile Gln Ala Leu Met Arg Glu Leu Glu Asp Gly Ser Pro Arg Ala Gly Gln Ile Leu
        +120                                                    +130


        420                 430                 440                 450                         460              470
AAG CAA ACC TAC GAC AAA TTT GAC ACA AAC TTG CGC AGT GAT GAC GCG CTG CTT AAG AAC
Lys Gln Thr Tyr Asp Lys Phe Asp Thr Asn Leu Arg Ser Asp Asp Ala Leu Leu Lys Asn
        +140                                                    +150


        480                 490                 500                 510                         520              530
TAC GGG CTG CTC TCC TGC TTC AAG AAG GAC CTG CAC AAG GCT GAG ACA TAC CTG CGG GTC
Tyr Gly Leu Leu Ser Cys Phe Lys Lys Asp Leu His Lys Ala Glu Thr Tyr Leu Arg Val
        +160                                                    +170


        540                 550                 560                 570                         580
ATG AAG TGT CGC CGC TTC GTG GAG AGC AGC TGT GCC TCC TAG T T G C T G G G C A T C
Met Lys Cys Arg Arg Phe Val Glu Ser Ser Cys Ala Phe Tem
        +180                                                    +190


        590                 600                 610                         620
T C T G T T G C C C C T C C C C A G T A C C T C C C C T G A C C T G G A A A G T


        630                 640                 650                         660
G C C A C C C C A A T G C C T G C T G T C C T T T C C T A A T A A A A C A G G T


        670
T G C A T C G T (A)₁₀(C)₂₄-Pst
```

# FIG. 6

| Restriction Endonuclease | Sequence | Cut | Positions |
|---|---|---|---|
| AluI | AGCT | Blunt | 144 218 295 332 374 559 |
| HaeI | AGGCCA | | 399 |
| TthIII-II | CAAPCC | | 415 |
| PvuII | CAGCTG | Blunt | 558 |
| BstNI | CCAGG | | 357 |
| AvaI/SmaI/XmaI | CCCGGG | | 392 |
| HpaII/MspI | CCGG | | 45 109 172 393 |
| MnlI | CCTC | | 273 311 408 566 595 606 |
| BstNI | CCTGG | | 342 616 |
| FnuDII | CGCG | Blunt | 97 319 456 |
| DdeI | CTNAG | | 274 312 512 |
| HsaI | GACGCNNNNNN | | 454 |
| TthIII-I | GACNNNGTC | | 316 |
| MnlI | GAGG | | 112 120 193 346 349 379 |
| MboI/Sam3aI | GATC | | 168 206 405 |
| SfaNI | GATGC | | 366 |
| HinfI | GANTC | | 165 |
| BbvI | GCAGC | | 386 557 |
| HhaI | GCGC | | 96 98 225 444 457 |
| HsaI | GCGTC | | 320 |
| BbvI | GCTGC | | 70 145 219 222 234 237 459 477 |
| AvaII | GGACC | | 339 498 |
| HaeIII | GGCC | Blunt | 48 174 195 260 360 400 |
| Sau96I | GGNCC | | 47 259 |
| RsaI | GTAC | Blunt | 123 603 |
| AccI | GTCTAC | | 322 |
| HsIaI | GTGCTC | | 40 |
| HphI | TCACC | | 287 |
| MboII | TCTTC | | 284 |
| MstI | TGCGCA | | 443 |

# FIG.7

# FIG. 8

pBGH-Δ9(Ser)

```
               10   11   12   13   14   15   16              187 188 189 190 191
          Met  Ser  Leu  Phe  Ala  Asn  Ala  Val  Leu       Ser Ser Cys Ala Phe

──────────ATG AGC TTG TTT GCC AAC GCT GTG CTC ─────────── GCC AGC TGT GCC TTC TAG ──────────
                                      ‾HgiAI‾                   ‾PvuII‾
```

pSGH-9D

```
            1    2    3    4    5    6    7    8    9   10   11   12   13   14   15        186 187 188 189 190
          Phe  Pro  Ala  Met  Pro  Leu  Ser  Ser  Leu  Phe  Ala  Asn  Ala  Val  Leu      Ser Ser Cys Ala Phe

──────────TTC CCA GCC ATG CCC TTG TCC AGC CTA TTT GCC AAC GCC GTG CTC ───────────── AGC AGC TGT GCC TTC TAG ──────────
                                                           ‾HgiAI‾                     ‾PvuII‾
```

pSGH-Δ7

```
            8    9   10   11   12   13   14   15              186 187 188 189 190
          Met  Ser  Leu  Phe  Ala  Asn  Ala  Val  Leu        Ser Ser Cys Ala Phe

──────────ATG AGC TTG TTT GCC AAC GCT GTG CTC ──────────── GCC AGC TGT CGC TTC TAG ──────────
```

```
differences
in SGH          C  A              C
coding sequence
```

# FIG.9

T4 DNA polymerase
w/3' exo activity single stranded
dATP 200 µM
5' → 3' polymerase x 200

(1) S1

ligate

# FIG. 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | JOURNAL OF BIOLOGICAL CHEMISTRY. vol. 245, no. 13, 10 July 1970, BALTIMORE US pages 3407 - 3415; MILLS, J.B. ET AL.: 'Cyanogen bromide cleavage and partial amino acid sequence of porcine growth hormone' * the whole document * --- | 1-10 | C12N15/18 C12P21/02 C07K13/00 A61K37/36 C12N1/21 //(C12N1/21, C12R1:19) |
| Y,D | GB-A-2 073 245 (YEDA RESEARCH AND DEVELOPMENT CO., LTD.) * the whole document * --- | 1-10 | |
| A,D | EP-A-0 047 600 (THE REGENTS OF THE UNIVERSITY OF CALIFORNIA) * the whole document * --- | 1-10 | |
| P,X | DNA vol. 2, no. 1, 1983, NEW YORK, US pages 37 - 45; SEEBURG, P.H. ET AL.: 'Efficient bacterial expression of bovine and porcine growth hormones' * the whole document * --- | 1-9 | TECHNICAL FIELDS SEARCHED (Int. Cl.4) |
| E | EP-A-0 111 389 (GENENTECH, INC.) * claims * ----- | 1-10 | C07K C12N C12P A61K A23K |

The present search report has been drawn up for all claims

| Place of search | Date of completion of the search | Examiner |
|---|---|---|
| THE HAGUE | 16 JANUARY 1992 | ANDRES S.M. |

EPO FORM 1503 03.82 (P0401)